**Europäisches Patentamt**

**European Patent Office**

**Office européen des brevets**

(19)

(11) **EP 1 429 588 A2**

(12) **EUROPEAN PATENT APPLICATION**

(43) Date of publication:
**16.06.2004 Bulletin 2004/25**

(51) Int Cl.⁷: $H05G\ 1/34$, H05G 1/28

(21) Application number: **03257441.0**

(22) Date of filing: **26.11.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **27.11.2002 JP 2002343649**

(71) Applicant: **GE Medical Systems Global Technology Company LLC**
**Waukesha, Wisconsin 53188-1696 (US)**

(72) Inventors:
• **Segawa, Koji**
**Hino-shi, Tokyo 191-8503 (JP)**
• **Sekiguchi, Junko**
**Hino-Shi, Tokyo 191-8503 (JP)**
• **Fujiya, Kyoko**
**Hino-shi, Tokyo 191-8503 (JP)**

(74) Representative: **Goode, Ian Roy**
**London Patent Operation**
**General Electric International, Inc.**
**15 John Adam Street**
**London WC2N 6LU (GB)**

(54) **X-ray controlling method and x-ray imaging apparatus**

(57) For the purpose of enabling reduction of exposure dose, in an X-ray controlling method for an X-ray imaging apparatus for producing an image based on detected X-ray signals, an upper limit of an X-ray exposure dose to a subject to be imaged is set (602), and the tube current of an X-ray tube is modulated so that the exposure dose does not exceed the upper limit (606 - 610). The modulation of the tube current is achieved by finding an exposure dose predicted value based on an imaging protocol, and modifying a tube current set value in the imaging protocol when the predicted value exceeds the upper limit.

**FIG. 11**

EP 1 429 588 A2

Printed by Jouve, 75001 PARIS (FR)

**Description**

The present invention relates to an X-ray controlling method and an X-ray imaging apparatus, and more particularly to a method of controlling the tube current of an X-ray tube, and an X-ray imaging apparatus for conducting imaging while controlling the tube current of an X-ray tube.

In conventional X-ray CT (computed tomography) apparatuses, the tube current of an X-ray tube is set before starting imaging as described in Japanese Patent Application Laid Open No. 2001-43993.

Although a subject to be imaged is desirably exposed to the lowest possible X-ray dose, the setting of the tube current in the above manner primarily stresses image quality in imaging, and does not necessarily stress reduction of exposure dose.

It is therefore an object of the present invention to provide an X-ray controlling method that enables reduction of exposure dose, and an X-ray imaging apparatus that conducts X-ray control by such method.

The present invention, in accordance with one aspect for solving the aforementioned problem, is an X-ray controlling method for an X-ray imaging apparatus for projecting X-rays from an X-ray tube onto a subject to be imaged and detecting transmitted X-rays, and producing an image based on detected X-ray signals, said method characterized in comprising: setting an upper limit of an X-ray exposure dose to the subject to be imaged; and modulating the tube current of the X-ray tube so that the exposure dose does not exceed the upper limit.

The present invention, in accordance with another aspect for solving the aforementioned problem, is an X-ray imaging apparatus for projecting X-rays from an X-ray tube onto a subject to be imaged and detecting transmitted X-rays, and producing an image based on detected X-ray signals, said apparatus characterized in comprising: setting means for setting an upper limit of an X-ray exposure dose to the subject to be imaged; and modulating means for modulating the tube current of the X-ray tube so that the exposure dose does not exceed the upper limit.

In the invention of the aspects as described in (1) and (2), an upper limit of an X-ray exposure dose is set for a subject to be imaged, and the tube current of the X-ray tube is modulated so that the exposure dose does not exceed the upper limit; and therefore, the exposure dose to the subject to be imaged is reduced.

Preferably, the X-ray imaging apparatus is an X-ray CT apparatus so that a tomographic image may be captured at a low exposure dose. Preferably, the X-ray CT apparatus conducts imaging by a helical scan so that a tomographic image over a wide range may be captured at a low exposure dose.

Preferably, the modulation of the tube current is achieved by: finding an exposure dose predicted value based on an imaging protocol; and modifying the tube current set value in the imaging protocol when the predicted value exceeds the upper limit, so that the tomographic imaging at a low exposure dose may be suitably conducted.

Preferably, the tube current set value is specified for each slice position so that the quality of tomographic images may be kept constant regardless of the slice position. Preferably, the modification is achieved by: modifying a tube current set value I to

$$I' = I \cdot (Du/Dc)^{1/2},$$

where the predicted value is denoted by Dc, and the upper limit is denoted by Du, so that the image SD of tomographic images may be kept constant regardless of the slice position.

The present invention, in accordance with still another aspect for solving the aforementioned problem, is an X-ray imaging apparatus for projecting X-rays from an X-ray tube onto a subject to be imaged and detecting transmitted X-rays, and producing an image based on detected X-ray signals, said apparatus characterized in comprising: calculating means for calculating a historical X-ray exposure dose to the subject to be imaged; and display means for displaying the calculated exposure dose.

In the invention of this aspect, since a historical X-ray exposure dose to a subject to be imaged is calculated by the calculating means and the calculated exposure dose is displayed by the display means, the exposure dose during new imaging can be reduced.

Preferably, the calculating means calculates the exposure dose based on historical imaging data for the subject to be imaged so that the exposure dose may be correctly calculated. Preferably, the calculating means acquires the historical imaging data from a server so that data acquisition may be facilitated. Preferably, the X-ray imaging apparatus is an X-ray CT apparatus so that the exposure dose during tomographic imaging may be reduced.

Therefore, the present invention provides an X-ray controlling method that enables reduction of exposure dose, and an X-ray imaging apparatus that conducts X-ray control by such method.

Further objects and advantages of the present invention will be apparent from the following description of the preferred embodiments of the invention as illustrated in the accompanying drawings, in which:

Figure 1 is a block diagram of an apparatus in accordance with one embodiment of the present invention.

Figure 2 is a schematic view of an X-ray detector.

Figure 3 is a schematic view of an X-ray detector.

Figure 4 is a schematic view of an X-ray emitting/detecting apparatus.

Figure 5 is a schematic view of the X-ray emitting/detecting apparatus.

Figure 6 is a schematic view of the X-ray emitting/detecting apparatus.

Figure 7 is a conceptual diagram of scout imaging.

Figure 8 is a graph showing a relationship between an oval ratio and an SD ratio.

Figure 9 is a flow chart of an operation of the apparatus in accordance with one embodiment of the present invention.

Figure 10 is a diagram showing a relationship between an X-ray impinging position on a body axis and a tube current.

Figure 11 is a flow chart of an operation of the apparatus in accordance with one embodiment of the present invention.

Figure 12 is a block diagram of an apparatus in accordance with one embodiment of the present invention.

Figure 13 is a block diagram of a medical image network.

Figure 14 is a flow chart of an operation of the apparatus in accordance with one embodiment of the present invention.

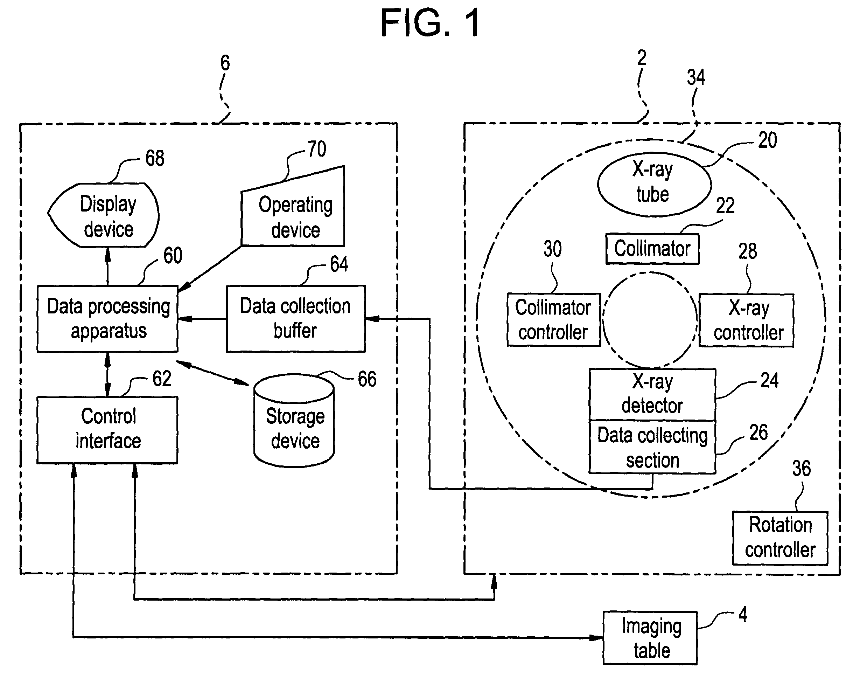

Several embodiments of the present invention will now be described in detail with reference to the accompanying drawings. Figure 1 shows a block diagram of an X-ray CT apparatus, which is an embodiment of the present invention. The configuration of the apparatus represents an embodiment of the apparatus in accordance with the present invention. The operation of the apparatus represents an embodiment of the method in accordance with the present invention.

As shown in Figure 1, the apparatus comprises a scan gantry 2, an imaging table 4 and an operating console 6. The scan gantry 2 has an X-ray tube 20. X-rays (not shown) emitted from the X-ray tube 20 are formed into a fan-shaped X-ray beam, i.e., a fan beam, by a collimator 22, and projected toward an X-ray detector 24.

The X-ray detector 24 has a plurality of detector elements arranged in line as an array in the extent direction of the fan-shaped X-ray beam. The configuration of the X-ray detector 24 will be described in detail later. The X-ray tube 20, collimator 22 and X-ray detector 24 together constitute an X-ray emitting/detecting apparatus, which will be described in detail later.

The X-ray detector 24 is connected with a data collecting section 26. The data collecting section 26 collects signals detected by the individual detector elements in the X-ray detector 24 as digital data.

The emission of the X-rays from the X-ray tube 20 is controlled by an X-ray controller 28. The interconnection between the X-ray tube 20 and X-ray controller 28 is omitted in the drawing. The collimator 22 is controlled by a collimator controller 30. The interconnection between the collimator 22 and collimator controller 30 is omitted in the drawing.

The above-described components from the X-ray tube 20 through the collimator controller 30 are mounted on a rotating section 34 of the scan gantry 2. The rotation of the rotating section 34 is controlled by a rotation controller 36. The interconnection between the rotating section 34 and rotation controller 36 is omitted in the drawing.

The imaging table 4 is configured to carry a subject to be imaged (not shown) into and out of an X-ray irradiation space in the scan gantry 2. The relationship between the subject and X-ray irradiation space will be described in detail later.

The operating console 6 has a data processing apparatus 60. The data processing apparatus 60 is comprised of, for example, a computer. The data processing apparatus 60 is connected with a control interface 62. The control interface 62 is connected with the scan gantry 2 and the imaging table 4. The data processing apparatus 60 controls the scan gantry 2 and imaging table 4 via the control interface 62.

The data collecting section 26, X-ray controller 28, collimator controller 30 and rotation controller 36 in the scan gantry 2 are controlled via the control interface 62. The individual connections between these sections and the control interface 62 are omitted in the drawing.

The data processing apparatus 60 is also connected with a data collection buffer 64. The data collection buffer 64 is connected with the data collecting section 26 in the scan gantry 2. Data collected at the data collecting section 26 are input to the data processing apparatus 60 via the data collection buffer 64.

The data processing apparatus 60 performs image reconstruction using transmitted X-ray data for a plurality of views collected via the data collection buffer 64. The image reconstruction is performed using a filtered backprojection technique, for example.

The data processing apparatus 60 is also connected with a storage device 66. The storage device 66 stores several kinds of data, programs, and so forth. Several kinds of data processing relating to imaging are achieved by the data processing apparatus 60 executing the programs stored in the storage device 66.

The data processing apparatus 60 is further connected with a display device 68 and an operating device 70. The display device 68 displays the reconstructed image and other information output from the data processing apparatus 60. The operating device 70 is operated by a user, and supplies several kinds of instructions and information to the data processing apparatus 60. The user interactively operates the present apparatus using the display device 68 and operating device 70.

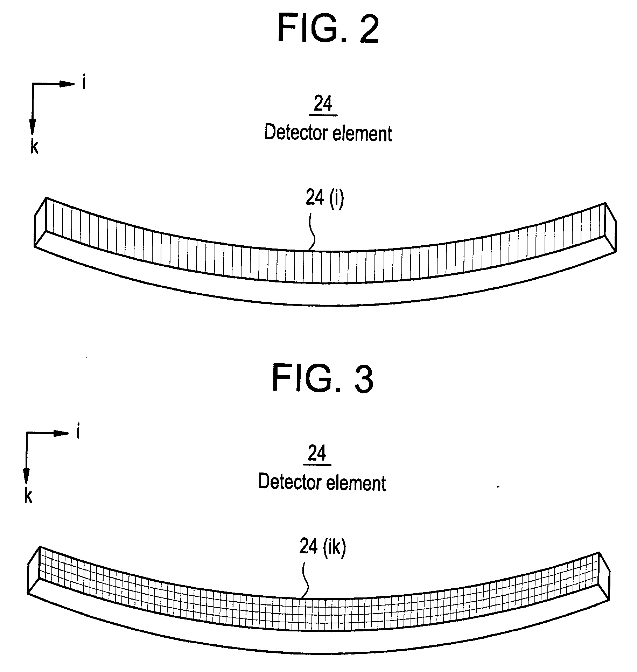

Figure 2 schematically shows one configuration of the X-ray detector 24. As shown, this X-ray detector 24 is a multi-channel X-ray detector having a multiplicity of detector elements 24(i) arranged in a one-dimensional array. Reference symbol 'i' designates a channel index and `i' = 1 — 1,000, for example. The detector elements 24(i) together form an X-ray impingement surface curved as a cylindrical concavity.

The X-ray detector 24 may instead be one having a plurality of detector elements 24(ik) arranged in a two-dimensional array, as shown in Figure 3. The detector elements 24(ik) together form an X-ray impingement surface curved as a cylindrical concavity. Reference symbol 'k' designates a row index and 'k' = 1, 2, 3, 4, for example. The detector elements 24(ik) that have the same row index 'k' together constitute a detector element row. The X-ray detector 24 is not limited to having four detector element rows, and may have a plurality of rows that is more or less than four rows.

Each detector element 24(ik) is formed of a combination of a scintillator and a photodiode, for example. It should be noted that the detector element 24(ik) is not limited thereto but may be a semiconductor detector element using cadmium telluride (CdTe) or the like, or an ionization chamber detector element using xenon (Xe) gas, for example.

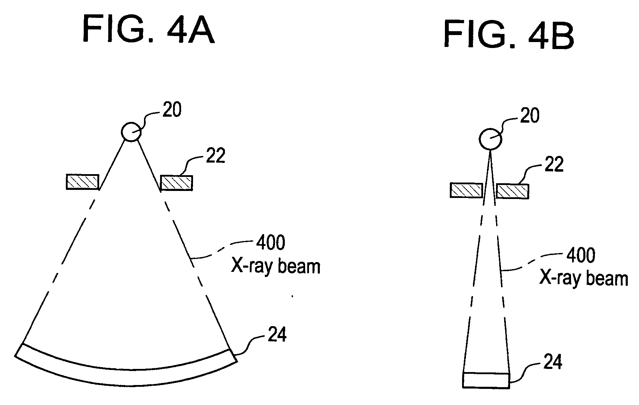

Figure 4 shows an interrelationship among the X-ray tube 20, collimator 22 and X-ray detector 24 in the X-ray emitting/detecting apparatus. Figure 4(a) is a view from the front of the scan gantry 2 and (b) is a view from the side thereof. As shown, the X-rays emitted from the X-ray tube 20 are formed into a fan-shaped X-ray beam 400 by the collimator 22, and projected toward the X-ray detector 24.

Figure 4(a) illustrates the extent of the fan-shaped X-ray beam 400. The extent direction of the X-ray beam 400 coincides with the direction of the linear arrangement of the channels in the X-ray detector 24. Figure 4(b) illustrates the thickness of the X-ray beam 400. The thickness direction of the X-ray beam 400 coincides with the direction of the side-by-side arrangement of the detector element rows in the X-ray detector 24.

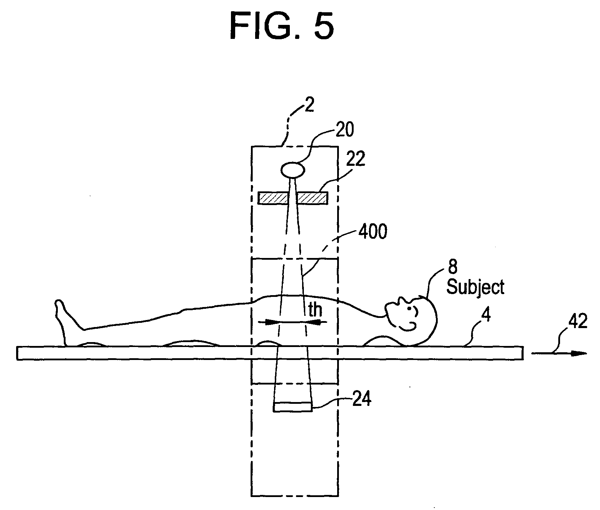

A subject 8 placed on the imaging table 4 is carried into the X-ray irradiation space with the subject's body axis intersecting the fan surface of such an X-ray beam 400, as exemplarily shown in Figure 5. The scan gantry 2 has a cylindrical structure containing therein the X-ray emitting/detecting apparatus.

The X-ray irradiation space is formed in the internal space of the cylindrical structure of the scan gantry 2. An image of the subject 8 sliced by the X-ray beam 400 is projected onto the X-ray detector 24. The X-rays passing through the subject 8 are detected by the X-ray detector 24. The thickness 'th' of the X-ray beam 400 impinging upon the subject 8 is regulated by the degree of opening of an aperture of the collimator 22.

The X-ray emitting/detecting apparatus comprised of the X-ray tube 20, collimator 22 and X-ray detector 24 continuously rotates (or scans) around the body axis of the subject 8 while maintaining their interrelationship. When the imaging table 4 is continuously moved in the body axis direction of the subject 8 as indicated by an arrow 42 simultaneously with the rotation of the X-ray emitting/detecting apparatus, the X-ray emitting/detecting apparatus will rotate relative to the subject 8 along a helical trajectory surrounding the subject 8, thus conducting a scan generally referred to as a helical scan. It will be easily recognized that when a scan is conducted with the imaging table 4 immobilized, a scan at a fixed slice position, i.e., an axial scan, is conducted.

Projection data for a plurality of (for example, ca. 1,000) views are collected per scan rotation. The collection of the projection data is conducted by a system including the X-ray detector 24, data collecting section 26 and data collection buffer 64.

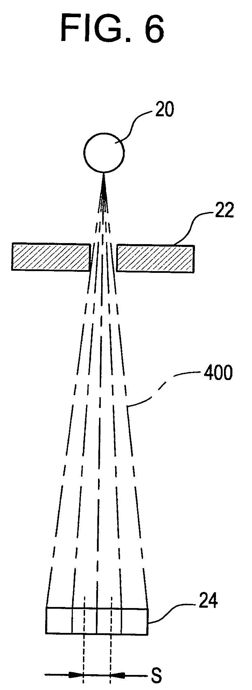

When the number of detector element rows in the X-ray detector 24 is four, data for four slices are simultaneously collected, as shown in Figure 6. The data processing section 60 uses the projection data for the four slices to perform image reconstruction.

Representing the distance between centers of adjacent slices as 's', and the movement distance of the X-ray emitting/detecting apparatus in the body axis direction per rotation of a helical scan as 'L', Us is generally referred to as the pitch of the helical scan.

Prior to such a scan, dose adjustment for the particular subject 8 is conducted. The dose adjustment is achieved by modulating the tube current-time product, i.e., the so-called milliampere-seconds (mAs), for the X-ray tube. The tube current-time product will be sometimes referred to simply as the tube current hereinbelow. Tube current adjustment for the particular subject 8 is sometimes referred to as auto-milliampere (auto mA).

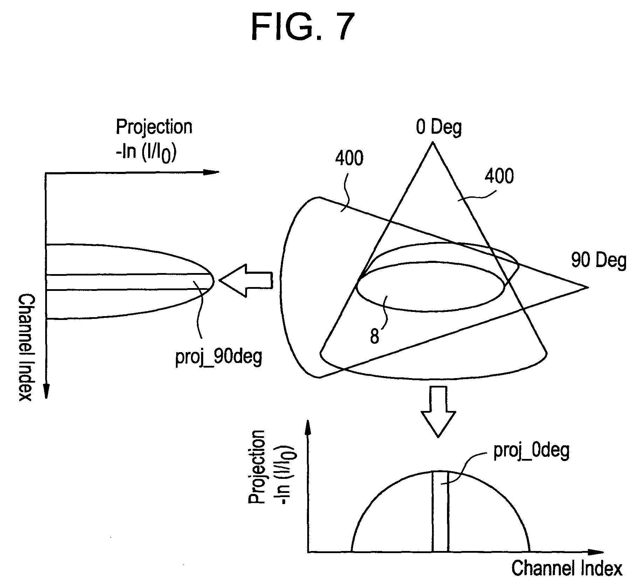

For the tube current adjustment, a projection of the subject 8 is measured. The measurement of the projection is achieved by fluoro-imaging the subject 8 by the X-ray beam 400 in, for example, a 0° (sagittal) direction and a 90° (lateral) direction, and obtaining respective projections, as conceptually shown in Figure 7. Such fluoro imaging will be sometimes referred to as scout imaging hereinbelow.

For these projections, respective projection areas are calculated by the equations below. The calculation is conducted by the data processing section 60. The same applies to the following description.

$$projection\_area = \sum_{i=1}^{i=max\_ch} proj_{0\deg i}, \tag{1}$$

and

$$projection\_area = \sum_{i=1}^{i=max\_ch} proj_{90\deg i}, \tag{2}$$

where

i: a channel index,

$proj_{0\deg i}$: projection data for each channel in the sagittal direction, and

$proj_{90\deg i}$: projection data for each channel in the lateral direction.

The projection areas calculated using Equations (1) and (2) will have the same value.

For the sagittal and lateral projections, respective center values are calculated using the following equations:

$$proj\_0\deg = \sum_{i=cent-49}^{i=cent+50} proj_{0\deg i}, \tag{3}$$

and

$$proj\_90\deg = \sum_{i=cent-49}^{i=cent+50} proj_{90\deg i}, \tag{4}$$

where

cent + 50: a number obtained by adding 50 to the center channel index, and

cent - 49: a number obtained by subtracting 49 from the center channel index.

Proj_0deg will be sometimes referred to as a sagittal center value and proj_90deg as a lateral center value hereinbelow.

The center values are used to calculate an oval ratio when the cross section of the subject 8 is assumed to be elliptical. The oval ratio is given by the following equation:

$$oval\_ratio = \frac{\sum_{i=cent-49}^{i=cent+50} proj_{90\,deg\,i}}{\sum_{i=cent-49}^{i=cent+50} proj_{0\,deg\,i}} . \qquad (5)$$

It should be noted that the numerator and denominator of the oval ratio are set so that the oval ratio has a value no less than one. Therefore, if the sagittal center value is greater than the lateral center value as in the head, the sagittal center value is set at the numerator and the lateral center value is set at the denominator, contrary to the equation above. The one of the sagittal and lateral center values that has a larger value corresponds to the major axis of the ellipse, and the other that has a smaller value corresponds to the minor axis.

It also possible to obtain only one projection fluoro-imaged in either the sagittal or the lateral direction. In this case, the projection area is obtained from either Equation (1) or (2) depending upon the direction of the fluoro-imaging, and the center value of the projection is similarly obtained from either Equation (3) or (4) depending upon the direction of the fluoro-imaging.

The relationship among the projection area, sagittal center value and lateral center value is given by the following equation:

$$projection\_area = (proj\_0deg \times proj\_90deg) \times S + I, \qquad (6)$$

where

S: an oval coefficient, and

I: an oval offset.

Therefore, if any two of the projection area, sagittal center value and lateral center value are known, the remaining one value can be arithmetically determined.

When the projection area and one of the center values are known from fluoro-imaging in one of the directions, the other center value is determined by the following equation:

$$proj\_orthogonal = \frac{projection\_area - I}{proj\_measure \times S}, \qquad (7)$$

where

proj_measure: a center value known by measurement.

Therefore, when proj_measure is the sagittal center value, the oval ratio is given by:

$$oval\_ratio = \frac{proj\_orthogonal}{proj\_measure}, \qquad (8)$$

and when proj_measure is the lateral center value, it is given by:

$$oval\_ratio = \frac{proj\_measure}{proj\_orthogonal} \qquad (9)$$

It will be easily recognized that also in this case, the numerator and denominator are set so that the oval ratio is no less than one.

The quality of a reconstructed image is represented by an image SD (image standard deviation). The image SD when the subject has a circular cross section is a function of the projection area under a certain reference dose, and is given by the following equation:

$$image\_SD = \alpha + \beta \times projection\_area + \gamma \times projection\_area^2, \tag{10}$$

where

$\alpha$, $\beta$, $\gamma$: constants that depend upon the tube voltage (kV) etc.

When the subject has an elliptical cross section, the image SD varies with the oval ratio. Assuming that the projection area is constant, the relationship between the oval ratio and the rate of change of the image SD is given by the following equation:

$$SD\_ratio = A + B \times oval\_ratio^2, \tag{11}$$

where

A, B: constants.

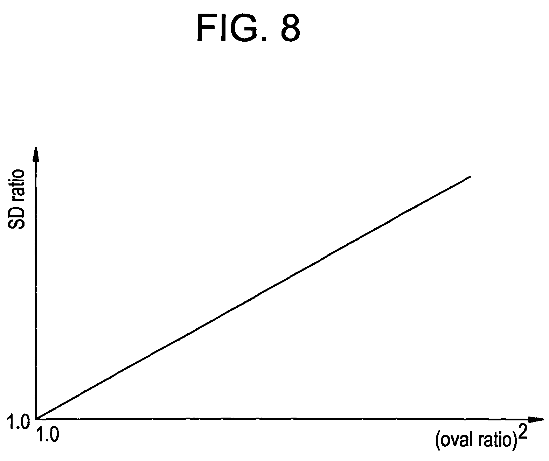

The relationship of Equation (11) is shown by the graph in Figure 8. As shown, when the oval_ratio is one, the SD ratio is one. That is, the image SD does not vary when the cross section is circular.
From such a relationship, when the subject has an elliptical cross section, a modified image SD is determined for the shape of the cross section by the following equation:

$$image\_SD' = image\_SD \times SD\_ratio. \tag{12}$$

The modified image SD is a predicted value for the image SD of a reconstructed image when the subject 8 is imaged by a reference dose. Since a target value of the image SD for the reconstructed image is determined beforehand, the dose must be set so that an image satisfying the target value is obtained.
The relationship among the image SD predicted value and the reference dose, and the image SD target value and the required dose is given by the following equation:

$$\frac{image\_SD_{target}}{image\_SD_{predited}} = \sqrt{\frac{mAs_{reference} \times thickness\_factor}{mAs_{scan}}}, \tag{13}$$

where

$image\_SD_{target}$: the image SD target value,

$image\_SD$ predicted: the image SD predicted value (= image_SD'),

$mAs_{reference}$: the reference dose,

$mAs_{scan}$: the required dose, and

$$thickness\_factor = \frac{10.0}{thickness(mm)} \tag{14}$$

The 'thickness' is the thickness of the X-ray beam 400 at the iso-center of the subject 8.
From Equation (13), the required dose is obtained as follows:

$$mAs_{scan} = \frac{mAs_{reference} \times thickness\_factor}{\left(\frac{image\_SD_{target}}{image\_SD_{predicted}}\right)^2}, \tag{15}$$

Thus, the tube current corresponding to the required dose is obtained as follows:

$$mA_{scan} = \frac{mAs_{scan}}{scan\_time(\sec)},$$ (16)

where 'scan_time' is the scan time of the present apparatus, i.e., the time period during one rotation of the X-ray emitting/detecting apparatus.

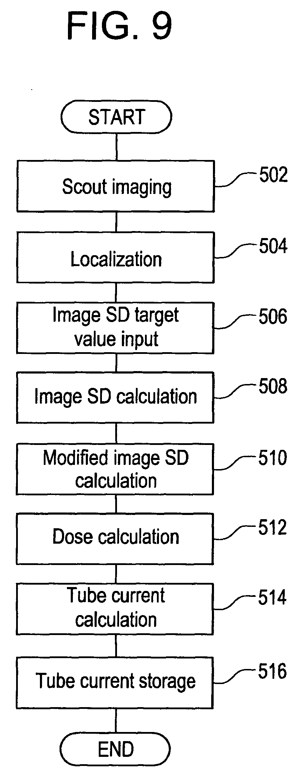

Figure 9 shows a flow chart of the operation from the scout imaging to the tube current calculation as described above. As shown, scout imaging is conducted at Step 502. By the scout imaging, the subject 8 is fluoro-imaged in one or both of sagittal and lateral directions over a certain range in the body axis direction, and respective projections at positions on the body axis are acquired.

Next, at Step 504, localization is conducted. The localization is for specifying scan start and end points on the body axis in a fluoroscopic image obtained by the scout imaging. This determines the length of the imaged range, and for a helical scan, determines a scan position for every pitch. The localization is performed by the user via the operating device 70.

Next, at Step 506, an image SD target value is input. The input is also performed by the user via the operating device 70. If a standard value prestored in the present apparatus is used for the image SD target value, input by default is possible.

Next, at Step 508, an image SD is calculated. In calculating the image SD, a projection area is first obtained. When the scout imaging is conducted in the sagittal and lateral directions, respective projection areas are obtained from Equations (1) and (2); and when the scout imaging is conducted in one of these directions, a projection area is obtained by Equation (1) or (2) depending upon the direction. The image SD is then calculated from Equation (10) using the projection area(s). The image SD is calculated for every pitch of the helical scan. Calculations for values described below are also conducted in the same way.

Next, at Step 510, a modified image SD is calculated. Prior to calculating the modified image SD, sagittal and lateral center values are obtained from Equations (3) and (4), respectively, and an oval ratio is obtained from Equation (5). Alternatively, a sagittal or lateral center value is obtained from Equation (3) or (4), a lateral or sagittal center value is obtained from Equation (7), and an oval ratio is obtained from Equation (8) or (9). An SD ratio is obtained from Equation (11) using the oval ratio, and the SD ratio is used to calculate the modified image SD from Equation (12).

Next, at Step 512, dose is calculated. The dose calculation is performed according to Equation (15). In this equation, the image SD target value input at Step 506 is used as $image\_SD_{target}$, and the two modified image SD's obtained as described above are used as image_SD predicted Thus, two doses are calculated.

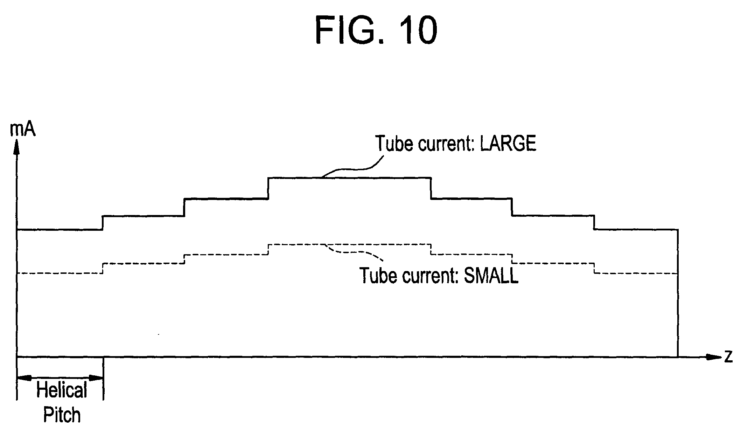

Next, at Step 514, a tube current is calculated. The tube current calculation is performed according to Equation (16). The solid line in Figure 10 shows an exemplary tube current thus calculated. As shown, the tube current is obtained for every position on the body axis. The tube current indicated by the broken line will be explained later. Next, at Step 516, the calculated values for the tube current are stored in the memory. Thus, the tube current is stored for every pitch of the helical scan.

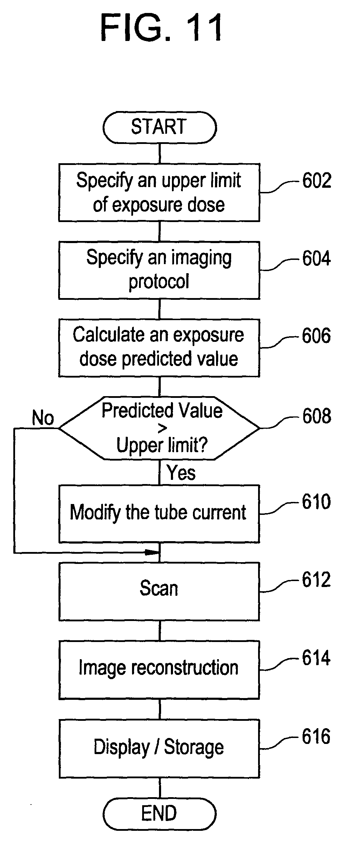

A general operation of the present apparatus will now be described. Figure 11 is a flow chart of the general operation of the present apparatus. As shown, at Step 602, an upper limit of the exposure dose is specified. The specification is performed by the user via the display device 68 and operating device 70. A portion comprised of the display device 68 and operating device 70 is an embodiment of the setting means of the present invention.

The exposure dose is set using a DLP (dose length product). The unit for the DLP is mGy . cm (milligray . centimeter). The upper limit for the DLP is specified as, for example, 300 mGy . cm. The upper limit for the DLP will be sometimes referred to as DLPu hereinbelow.

Next, at Step 604, an imaging protocol is specified. When auto-milliampere is employed, the protocol specification is achieved by the operation as shown in Figure 9. By auto-milliampere, a tube current for the particular subject 8 is set. When auto-milliampere is not employed, the imaging protocol desired by the user is specified, and the tube current is also set at a desired value.

Next, at Step 606, a predicted value for the exposure dose is calculated. The calculation of the exposure dose predicted value is achieved based on the tube current set value. Specifically, based on the tube current set value, a CTDIvol (CT dose index volume) is first calculated. The calculation of the CTDIvol based on the tube current is executed using a predefined algorithm.

Alternatively, the CTDIvol is found from a pre-measured relationship between the CTDIvol and tube current using a phantom, for example. The unit for the CTDIvol is mGy. The exposure dose predicted value is obtained by multiplying the CTDIvol by the imaged length in the body axis direction. The exposure dose predicted value will be denoted by DLPc hereinbelow.

It should be noted that the upper limit for the exposure dose may be set by the CTDIvol rather than the DLP.

In this case, the exposure dose predicted value is also obtained as the CTDlvol. The upper limit and predicted value for the CTDlvol will be denoted by CTDlvolu and CTDlvolc, respectively, hereinbelow.

Next, at Step 608, a decision is made on whether the predicted value is greater than the upper limit. If the predicted value is greater than the upper limit, the tube current is modified at Step 610. When the tube current has been set by auto-milliampere, the modification of the tube current is performed according to the following equation:

$$I' = I \cdot (DLPu/DLPc)^{1/2}$$

or

$$I' = I \cdot (CTDIvolu/CTDIvolc)^{1/2},$$

where I is the tube current set by auto-milliampere, i.e., an unmodified tube current, and I' is the modified tube current. By such modification, the tube current by auto-milliampere as indicated by the solid line in Figure 10 is modified into one as indicated by the broken line in Figure 10, for example.

If the tube current I has been set without using auto-milliampere, the tube current modification is achieved as follows: the upper limit DLPu is divided by the imaged length to obtain the CTDlvol, and the tube current is obtained based on the CTDlvol by inverting the aforementioned algorithm. Alternatively, the tube current may be obtained based on the pre-measured relationship between the CTDlvol and tube current. If the upper limit is set using the CTDlvol, the tube current can be obtained from the CTDlvol without the division by the imaged length. The data processing apparatus 60 for conducting the processing of Steps 606 - 610 is an embodiment of the modulating means of the present invention.

The tube current modification may alternatively be conducted using the following equation. This facilitates the tube current modification.

$$I' = I \cdot (DLPu/DLPc)$$

or

$$I' = I \cdot (CTDIvolu/CTDIvolc).$$

Next, at Step 612, a scan is conducted. The scan uses the tube current modified as described above. This allows a scan to be conducted without the exposure dose exceeding the upper limit. If the predicted value does not exceed the upper limit, the unmodified tube current is used to achieve a scan without the exposure dose exceeding the upper limit. Next, at Step 614, image reconstruction is conducted. A reconstructed image is displayed on the display device 68 and stored in the memory at Step 616.

Although the preceding description has been made on a case in which a helical scan is conducted, it will be easily recognized that the present technique is not limited to the case of a helical scan but also enables a similar effect to be obtained in conducting an axial scan.

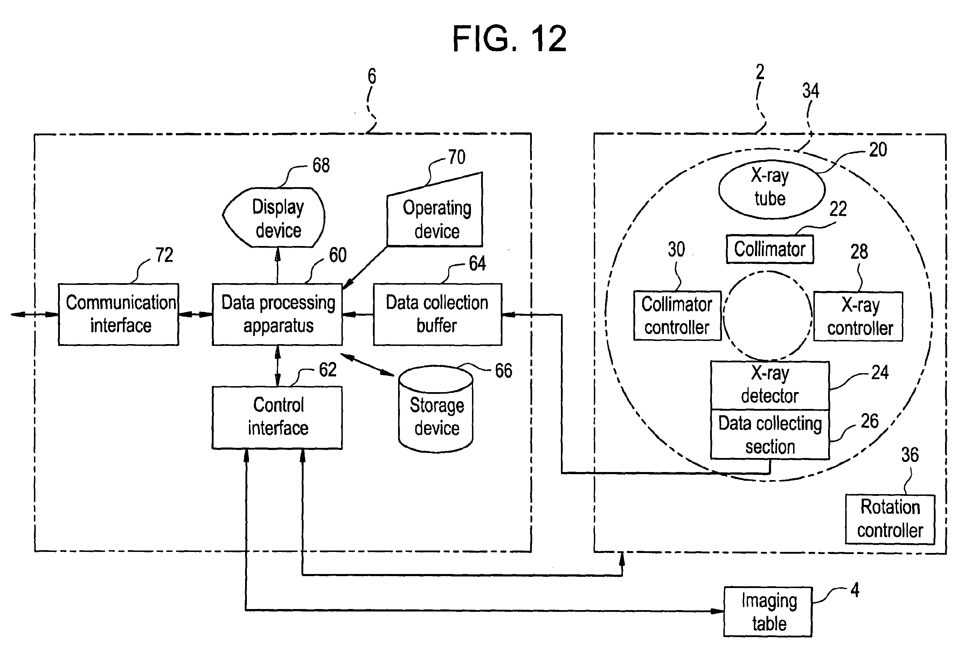

Figure 12 shows a block diagram of an X-ray CT apparatus, which is an embodiment of the present invention. The configuration of the apparatus represents an embodiment of the apparatus in accordance with the present invention. In Figure 12, parts similar to those shown in Figure 1 are designated by similar reference numerals, and explanation thereof will be omitted.

The present apparatus comprises a communication interface 72. The communication interface 72 is disposed between an external communication network and the data processing apparatus 60. The data processing apparatus 60 exchanges data with the outside via the communication interface 72.

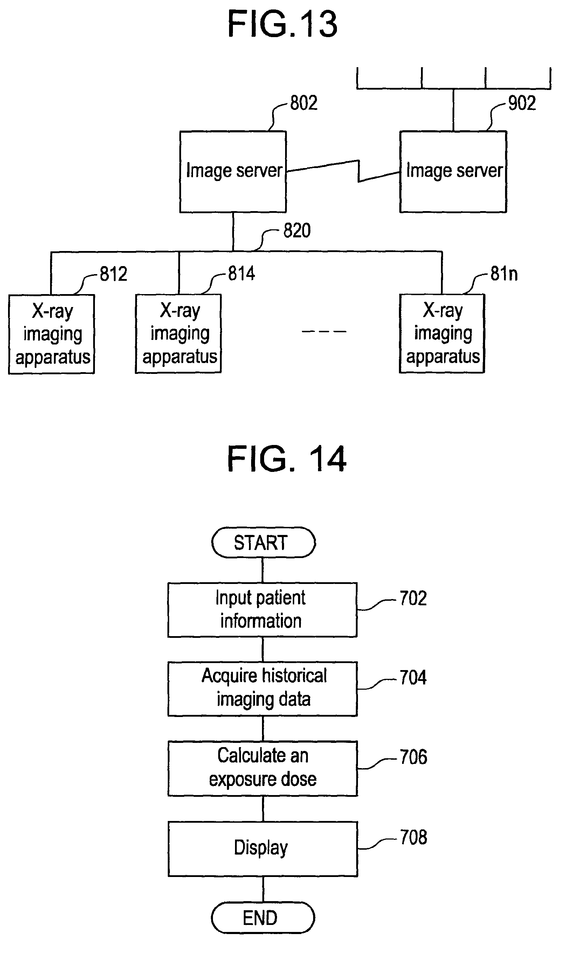

Figure 13 shows a block diagram of a medical image network to which the present apparatus belongs. As shown, an image server 802 and a plurality of X-ray imaging apparatuses 812, 814, ..., 81n are connected via a communication circuit 820 to constitute a medical image network. The X-ray imaging apparatus 81 i (i: 2, 4, ..., n) is an X-ray CT apparatus, for example. However, the X-ray imaging apparatus is not limited to the X-ray CT apparatus but may be an appropriate imaging apparatus conducting imaging using X-rays, such as an X-ray fluoroscopic imaging apparatus.

The image server 802 archives images captured by each X-ray imaging apparatus 81i (i: 2, 4, ..., n) and associated information. The information is accessible by each X-ray imaging apparatus 81i. If another image server 902 separate from the image server 802 is connected via the communication circuit, the X-ray imaging apparatus 81i can access the image server 902. The image servers 802 and 902 represent an embodiment of the server of the present invention.

The present apparatus is configured to be capable of accessing the image server 802 (or 902 or both) to acquire historical information on a particular patient, calculating an X-ray dose to which the patient has been exposed thus far based on the information, and displaying the X-ray dose on the display device 68.

Specifically, as shown in the flow chart of Figure 14, upon input of patient information at Step 702, the data processing apparatus 60 acquires historical imaging data at Step 704, and calculates an exposure dose at Step 706. Thus, the exposure dose over the past year is found, for example, and is displayed at Step 708.

The data processing apparatus 60 for executing the processing at Steps 704 and 706 is an embodiment of the calculating means of the present invention. The display device 68 for executing the display at Step 708 is an embodiment of the display means of the present invention.

Thus, the user of the present apparatus can know the exposure dose to date for a patient. The exposure dose may be effectively used as reference data for present or future imaging to reduce the total amount of the exposure dose to the patient.

For completeness, various aspects of the invention are set out in the following numbered clauses:

1. An X-ray controlling method for an X-ray imaging apparatus for projecting X-rays from an X-ray tube onto a subject to be imaged and detecting transmitted X-rays, and producing an image based on detected X-ray signals, comprising the steps of:

setting an upper limit of an X-ray exposure dose to the subject to be imaged; and
modulating the tube current of the X-ray tube so that the exposure dose does not exceed the upper limit.

2. The X-ray controlling method of clause 1, wherein said X-ray imaging apparatus is an X-ray CT apparatus.

3. The X-ray controlling method of clause 2, wherein said X-ray CT apparatus conducts imaging by a helical scan.

4. The X-ray controlling method of clause 2, wherein said step of modulating the tube current is achieved by: finding an exposure dose predicted value based on an imaging protocol; and modifying the tube current set value in the imaging protocol when the predicted value exceeds said upper limit.

5. The X-ray controlling method of clause 4, wherein said tube current set value is specified for each slice position.

6. The X-ray controlling method of clause 5, wherein said step of modulation is achieved by modifying a tube current set value I to

$$I' = I \cdot (Du/Dc)^{1/2},$$

where said predicted value is denoted by Dc, and said upper limit is denoted by Du.

7. An X-ray imaging apparatus for projecting X-rays from an X-ray tube onto a subject to be imaged and detecting transmitted X-rays, and producing an image based on detected X-ray signals, comprising:

a setting device for setting an upper limit of an X-ray exposure dose to the subject to be imaged; and
a modulating device for modulating the tube current of the X-ray tube so that the exposure dose does not exceed the upper limit.

8. The X-ray imaging apparatus of clause 7, wherein said X-ray imaging apparatus is an X-ray CT apparatus.

9. The X-ray imaging apparatus of clause 8, wherein said X-ray CT apparatus conducts imaging by a helical scan.

10. The X-ray imaging apparatus of clause 8, wherein said modulating device finds an exposure dose predicted value based on an imaging protocol, and modifies the tube current set value in the imaging protocol when the predicted value exceeds said upper limit.

11. The X-ray imaging apparatus of clause 10, wherein said tube current set value is specified for each slice position.

12. The X-ray imaging apparatus of clause 11, wherein said modulating device modifies a tube current set value I to

$$I' = I \cdot (Du/DC)^{1/2},$$

where said predicted value is denoted by Dc, and said upper limit is denoted by Du.

13. An X-ray imaging apparatus for projecting X-rays from an X-ray tube onto a subject to be imaged and detecting transmitted X-rays, and producing an image based on detected X-ray signals, comprising:

a calculating device for calculating a historical X-ray exposure dose to the subject to be imaged; and
a display device for displaying the calculated exposure dose.

14. The X-ray imaging apparatus of clause 13, wherein said calculating device calculates the exposure dose based on historical imaging data for the subject to be imaged.

15. The X-ray imaging apparatus of clause 14, wherein said calculating device acquires the historical imaging data from a server.

16. The X-ray imaging apparatus of clause 13, wherein said X-ray imaging apparatus is an X-ray CT apparatus.

## Claims

1. An X-ray controlling method for an X-ray imaging apparatus for projecting X-rays from an X-ray tube onto a subject to be imaged and detecting transmitted X-rays, and producing an image based on detected X-ray signals, comprising the steps of:

setting an upper limit of an X-ray exposure dose to the subject to be imaged; and
modulating the tube current of the X-ray tube so that the exposure dose does not exceed the upper limit.

2. The X-ray controlling method of claim 1, wherein said X-ray imaging apparatus is an X-ray CT apparatus.

3. The X-ray controlling method of claim 2, wherein said X-ray CT apparatus conducts imaging by a helical scan.

4. The X-ray controlling method of claim 2, wherein said step of modulating the tube current is achieved by: finding an exposure dose predicted value based on an imaging protocol; and modifying the tube current set value in the imaging protocol when the predicted value exceeds said upper limit.

5. An X-ray imaging apparatus for projecting X-rays from an X-ray tube onto a subject to be imaged and detecting transmitted X-rays, and producing an image based on detected X-ray signals, comprising:

a setting device for setting an upper limit of an X-ray exposure dose to the subject to be imaged; and
a modulating device for modulating the tube current of the X-ray tube so that the exposure dose does not exceed the upper limit.

6. The X-ray imaging apparatus of claim 5, wherein said X-ray imaging apparatus is an X-ray CT apparatus.

7. The X-ray imaging apparatus of claim 6, wherein said X-ray CT apparatus conducts imaging by a helical scan.

8. The X-ray imaging apparatus of claim 6, wherein said modulating device finds an exposure dose predicted value based on an imaging protocol, and modifies the tube current set value in the imaging protocol when the predicted value exceeds said upper limit.

9. An X-ray imaging apparatus for projecting X-rays from an X-ray tube onto a subject to be imaged and detecting transmitted X-rays, and producing an image based on detected X-ray signals, comprising:

a calculating device for calculating a historical X-ray exposure dose to the subject to be imaged; and

EP 1 429 588 A2

a display device for displaying the calculated exposure dose.

10. The X-ray imaging apparatus of claim 9, wherein said calculating device calculates the exposure dose based on historical imaging data for the subject to be imaged.

FIG. 1

# FIG. 2

i →

↓ k

**24**
Detector element

24 (i)

# FIG. 3

i →

↓ k

**24**
Detector element

24 (ik)

EP 1 429 588 A2

## FIG. 4A

20
22
400
X-ray beam
24

## FIG. 4B

20
22
400
X-ray beam
24

# FIG. 5

# FIG. 6

# FIG. 7

Projection
-ln (I/I$_0$)

Channel Index

proj_90deg

0 Deg

400

400

90 Deg

8

Projection
-ln (I/I$_0$)

proj_0deg

Channel Index

# FIG. 8

# FIG. 9

START

Scout imaging — 502

Localization — 504

Image SD target value input — 506

Image SD calculation — 508

Modified image SD calculation — 510

Dose calculation — 512

Tube current calculation — 514

Tube current storage — 516

END

EP 1 429 588 A2

# FIG. 10

mA

Tube current: LARGE

Tube current: SMALL

Z

Helical
Pitch

# FIG. 11

START

Specify an upper limit of exposure dose — 602

Specify an imaging protocol — 604

Calculate an exposure dose predicted value — 606

Predicted Value > Upper limit? — 608

No

Yes

Modify the tube current — 610

Scan — 612

Image reconstruction — 614

Display / Storage — 616

END

# FIG. 12

Communication interface — 72

Display device — 68

Operating device — 70

Data processing apparatus — 60

Data collection buffer — 64

Control interface — 62

Storage device — 66

X-ray tube — 20

Collimator — 22

Collimator controller — 30

X-ray controller — 28

X-ray detector — 24

Data collecting section — 26

Rotation controller — 36

Imaging table — 4

6

2

34

EP 1 429 588 A2

# FIG.13

Image server ~802

Image server ~902

~820

X-ray imaging apparatus ~812

X-ray imaging apparatus ~814

— — —

X-ray imaging apparatus ~81n

# FIG. 14

START

Input patient information ~702

Acquire historical imaging data ~704

Calculate an exposure dose ~706

Display ~708

END